# EUROPEAN PATENT APPLICATION

(11) **EP 4 570 266 A1**
(43) Date of publication of application: **18.06.2025**
(21) Application number: 23216103.4
(22) Date of filing: 13.12.2023
(51) Int. Cl.: A61K 39/395, A61P 9/00, A61P 37/06, C07K 16/40

(54) **ANTIBODY TARGETING EXTRACELLULAR CYCLOPHILIN A**

(71) Applicant: Eberhard Karls Universität Tübingen Medizinische Fakultät, 72074 Tübingen (DE)
(72) Inventor: Gawaz, Meinrad, 72076 Tübingen (DE); Heinzmann, David, 72020 Tübingen (DE); Sigle, Manuel, 72076 Tübingen (DE)
(74) Representative: Witte, Weller & Partner Patentanwälte mbB

(57) **Abstract**

The present invention relates to an antibody or fragment thereof specifically binding to extracellular cyclophilin A (eCyPA), an antibody or fragment thereof for use in the prophylaxis and/or treatment of a systemic disease, preferably an inflammatory systemic disease such as a cardiovascular disease, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of a systemic disease, preferably an inflammatory systemic disease such as a cardiovascular disease, in a living being, comprising the administration of a prophylactically and/or therapeutically effective amount of said antibody or fragment thereof or said pharmaceutical composition.

## Description

The present invention relates to an antibody or fragment thereof, which specifically binds to extracellular cyclophilin A (eCyPA), an antibody or fragment thereof for use in the prophylaxis and/or treatment of a systemic disease, preferably an inflammatory systemic disease such as a cardiovascular disease, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of a systemic disease, preferably an inflammatory systemic disease such as a cardiovascular disease, in a living being, comprising the administration of a prophylactically and/or therapeutically effective amount of said antibody or fragment thereof or said pharmaceutical composition.

### BACKGROUND

Cardiac hypertrophy, characterized by an increase in heart size and mass, is a complex pathological condition that leads to heart failure and cardiovascular morbidity. Hypertrophy can be interpreted as a fundamental response of cardiac cells to internal or external stress, such as hypertension, valvular heart disease, cardiomyopathy, and myocardial infarction. One of the key features of cardiac hypertrophy is the remodeling of the myocardium, which involves alterations in the extracellular matrix (ECM) and cardiomyocyte structure. These changes contribute to myocardial stiffening, impaired relaxation, and contractile dysfunction, ultimately leading to a compromised cardiac function.

In recent years, emerging evidence has implicated that extracellular signaling molecules are critically involved in the pathogenesis of cardiac hypertrophy and remodeling. Among these molecules, cyclophilin A (CyPA), a highly conserved immunophilin, has gained attention due to its contrary intracellular and extracellular functions. CyPA is a ubiquitous protein that is predominantly located intracellularly, where it functions as a peptidyl-prolyl cis-trans isomerase, facilitating protein folding and trafficking, as well as signaling and proliferation. However, recent studies have demonstrated that CyPA can also be secreted into the extracellular milieu upon cell activation, hypoxia, or oxidative stress. There, it acts as a proinflammatory and profibrotic mediator via interaction with its receptor EMMPRIN (extracellular matrix metalloproteinase inducer, CD147). Disrupting intracellular activation of calcineurin and NFAT by administration of the CyPA-binding drug cyclosporine (CsA) has been extensively used in the past to block cytokine gene transcription. Although widely known for its immunosuppressive potential the role of CsA is discussed controversially. Side effects like increased susceptibility to infections and cancer as well as enhanced cardiovascular risk diminish the attractiveness of cyclosporine A as a viable drug for widespread clinical use. On the other hand, targeting inflammation by antagonizing interleukin-1 or using colchicine has been proposed to be a strategy to improve outcome in patients with ischemic heart failure.

Recent studies have suggested that the actual culprit of adverse cardiovascular effects may be mainly attributable to extracellular rather than intracellular CyPA. Therefore, it was proposed to inhibit eCyPA by a cyclosporin A derivative called 'MM284' as an approach to treat myocarditis; see Heinzmann et al. (2015), The novel extracellular cyclophilin A (CyPA) - inhibitor MM284 reduces myocardial inflammation and remodeling in a mouse model of troponin I -induced myocarditis, PLoS One 10(4): e0124606. However, this compound has not yet proven itself in practice and there is a need for new compounds with which inflammatory systemic diseases can be treated or prevented in a targeted manner.

### SUMMARY

Against this background, it is the object of the present invention to provide an alternative or new agent or compound with which the disadvantages of the prior art can be avoided or at least reduced. In particular, such an agent or compound is to be provided with which a systemic disease, preferably an inflammatory systemic disease such as a cardiovascular disease, can be prevented or treated in a targeted manner.

The problem underlying the invention is solved by the provision of an antibody or fragment thereof, which specifically binds to extracellular cyclophilin A (eCyPA), characterized in comprising
- as heavy chain variable domain a CDR3 region, said CDR3 region has an amino acid sequence being at least 90% identical to SEQ ID NO: 1, and/or
- as light chain variable domain a CDR3 region, said CDR3 region has an amino acid sequence being at least 90% identical to SEQ ID NO: 2.

The inventors were able to develop a neutralizing monoclonal antibody directed specifically and selectively against the extracellular species of CyPA (eCyPA). The inventors succeeded in demonstrating in a well-established animal model that the antibody-based inhibition of eCyPA prevents heart failure. Based on these findings the inventors identified the antigen-interacting sections of the said antibody and herewith provide a generic antibody or fragment thereof comprising the minimum of eCyPA binding and inhibiting domains, which can be successfully used for the treatment and prevention of systemic diseases.

The term "antibody" is intended to include any polypeptide chaincontaining molecular structure with a specific shape that fits to and recognizes an epitope, where one or more non-covalent binding interactions may stabilize the complex between the molecular structure and the epitope. The term includes both polyclonal and monoclonal antibodies. The archetypal antibody molecule is the immunoglobulin, and all types of immunoglobulins, IgG, IgM, IgA, IgE, IgD, etc., from all sources, e.g., human, rodent, rabbit, cow, sheep, pig, dog, camelid, other mammals, chicken, other avians, etc., are considered to be "antibodies". A preferred source for producing antibodies useful as starting material according to the invention is rabbits.

According to the invention, "CDR" refers to the complementary determining regions which, along with the framework regions (FR), are parts of the variable regions of the immunoglobulin and T cell receptor chains. CDRs and FRs are defined according to the International ImMunoGeneTics (IMGT) information system; see https://www.imgt.org/.

In an embodiment of the invention the antibody is a humanized antibody, i.e., an immunoglobulin or antibody that includes at least one humanized immunoglobulin or antibody chain (i.e., at least one humanized light or heavy chain). The term "humanized immunoglobulin chain" or "humanized antibody chain" (i.e., a "humanized immunoglobulin light chain" or "humanized immunoglobulin heavy chain") refers to an immunoglobulin or antibody chain (i.e., a light or heavy chain, respectively) having a variable region that includes a variable framework region substantially from a human immunoglobulin or antibody and complementarity determining regions (CDRs) (e.g., at least one CDR, preferably two CDRs, more preferably three CDRs) substantially from a non-human immunoglobulin or antibody, and further includes constant regions (e.g., at least one constant region or portion thereof, in the case of a light chain, and preferably three constant regions in the case of a heavy chain). The term "humanized variable region" (e.g., "humanized light chain variable region" or "humanized heavy chain variable region") refers to a variable region that includes a variable framework region substantially from a human immunoglobulin or antibody and complementarity determining regions (CDRs) substantially from a non-human immunoglobulin or antibody.

"Antibody fragments" comprise a portion of a full-length antibody, preferably the variable domain thereof, or at least the antigen binding site thereof, such as any of CDR3, CDR2 or CDR1. Examples of antibody fragments include diabodies, single-chain antibody molecules (scFv or scFab), and multispecific antibodies (e.g., bispecific) formed from antibody fragments.

The antibody or fragment thereof according to the invention can be produced by recombinant means. Methods for recombinant production are widely known in the state of the art and comprise protein expression in prokaryotic and eukaryotic cells with subsequent isolation of the antibody or fragment and usually purification to a pharmaceutically acceptable purity. For the expression of the antibodies or fragments as aforementioned in a host cell, nucleic acids encoding the respective light and/or heavy chains or fragments are inserted into expression vectors by standard methods. Expression is per-formed in appropriate prokaryotic or eukaryotic host cells like CHO cells, NSO cells, SP2/0 cells, HEK293 cells, COS cells, PER.C6 cells, yeast, or *E*. *coli* cells, and the anti-body or fragment is recovered from the cells (supernatant or cells after lysis). General methods for recombinant production of antibodies are well-known in the state of the art and described, for example, in the review articles of Makrides, S.C. (1999), Protein Expr. Purif. 17, 183-202; Geisse et al. (1996), Protein Expr. Purif. 8, 271-282; Kaufman, R.J. (2000), Mol. Biotechnol. 16, 151-161; Werner, R.G. (1998), J. Drug Res. 48, 870-880.

As used herein, the term "binding" or "specifically binding" refers to the binding of the antibody or fragment thereof to an epitope of the antigen (eCyPA), e.g., with purified wild-type eCyPA antigen in an *in vitro* assay such as a plasmon resonance assay (BIAcore, GE-Healthcare Uppsala, Sweden). Preferably, a specifically binding antibody or fragment does not exhibit significant cross-reactivity. An antibody or fragment thereof that "does not exhibit significant cross-reactivity" is one that will not appreciably bind to an undesirable entity, e.g., an undesirable proteinaceous entity. For example, an antibody or fragment that specifically binds to eCyPA will appreciably bind eCyPA but will not significantly react with non-eCyPA proteins or peptides, e.g., intracellular CyPA. An antibody specific for a preferred epitope will, for example, not significantly cross-react with remote epitopes on the same protein or peptide. Specific binding can be determined according to any art-recognized means for determining such binding. Preferably, specific binding is determined according to Scatchard analysis and/or competitive binding assays. The affinity of the binding is defined by the terms ka (rate constant for the association of the antibody from the antibody/antigen complex), kD (dissociation constant), and KD (kD/ka). Binding or specifically binding means a binding affinity (KD) of 10⁻⁸ mol/l or less, preferably 10⁻⁹ M to 10⁻¹³ mol/l.

In an embodiment of the invention the antibody or fragment thereof is isolated. The term "isolated" means that the material is removed from its original environment (e.g., the natural environment, if it is naturally occurring). For example, a naturally occurring antibody or fragment thereof present in a living animal is not isolated, but the same antibody or fragment thereof, separated from some or all of the coexisting materials in the natural system, is isolated.

The inventors have recognized that, in one embodiment of the invention, the antibodies or fragments thereof need not necessarily be sequence identical with the indicated sequences, e.g., of the CDRs or CDR3 respectively. Specific and affine binding of the antibody or fragment is also possible if the binding regions are at least 90% identical with the indicated specific sequences.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each amino acid or base in the Reference Sequence that does not have a corresponding aligned base or amino acid in the Compared Sequence and
(ii) each gap in the Reference Sequence and
(iii) each aligned amino acid or base in the Reference Sequence that is different from an aligned base or amino acid in the Compared Sequence, constitutes a difference and
(iv) the alignment has to start at position 1 of the aligned sequences;
and R is the number of amino acids or bases in the Reference Sequence over the length of the alignment with the Compared Sequence with any gap created in the Reference Sequence also being counted as an amino acid or base.

According to the invention, throughout the description and with respect to all embodiments, "at least 90%" identity includes a sequence identity of 90, 91, 92, 93, 94, 95, 96, 97, 98, 99, and 100%.

The antibody or fragment thereof disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from cellular material have been conventionally purified to electrophoretic homogeneity. Purification of starting material or natural material to at least one order of magnitude, preferably two or three orders, and more preferably four or five orders of magnitude is expressly contemplated. Furthermore, a claimed antibody or fragment thereof which has a purity of preferably 99.999%, or at least 99.99% or 99.9%; and even desirably 99% by weight or greater is expressly encompassed.

The antibody or fragment thereof according to the invention may be in "enriched form". As used herein, the term "enriched" means that the concentration of the material is at least about 2, 5, 10, 100, or 1000 times its natural concentration (for example), advantageously 0.01 %, by weight, preferably at least about 0.1 % by weight. Enriched preparations of about 0.5%, 1%, 5%, 10%, and 20% by weight are also contemplated. The sequences, constructs, vectors, cells, and other materials comprising the present invention can advantageously be in enriched or isolated form.

Cyclophilin A (CypA), also known as peptidylprolyl isomerase A (PPIA) or rotamase A is a member of the peptidyl-prolyl cis-trans isomerase (PPlase) family. It catalyzes the cis-trans isomerization of proline imidic peptide bonds, which allows it to regulate many biological processes, including intracellular signaling, transcription, inflammation, and apoptosis. It has been shown that cyclophilin A (CyPA) is involved in various pathophysiological mechanisms of cardiovascular diseases. CyPA can be secreted in the extracellular space (eCyPA) by inflammatory stimuli and is released upon cell death. Especially the interaction of eCyPA and the extracellular matrix metalloproteinase inducer (EMMPRIN, CD147) has been identified as an important factor in inflammatory processes such as leucocyte chemotaxis and induction of matrix metalloproteinases (MMP). In humans (Entrez: 5478, UniProt: P62937) CyPA is encoded by the *PPIA* gene on chromosome 7.

The findings of the inventors were surprising. For example, eCyPA has already been described in the prior art as a possible target for myocarditis therapy; cf. Heinzmann et al. (*I.c*.). However, it has not yet been possible to provide specific antibodies or antibody sequences that are suitable for such therapy.

In another embodiment of the invention said antibody or fragment thereof is further characterized in that
- the heavy chain variable domain comprises a CDR2 region, said CDR2 region has an amino acid sequence being at least 90% identical to SEQ ID NO: 3, and/or
- the light chain variable domain comprises a CDR2 region, said CDR2 region has an amino acid sequence being at least 90% identical to SEQ ID NO: 4.

In still another embodiment of the invention said antibody or fragment thereof is further characterized in that
- the heavy chain variable domain comprises a CDR1 region, said CDR1 region has an amino acid sequence being at least 90% identical to SEQ ID NO: 5, and/or
- the light chain variable domain comprises a CDR1 region, said CDR1 region has an amino acid sequence being at least 90% identical to SEQ ID NO: 6.

This measure provides the two further CDRs, i.e., CDR2 and CDR1, responsible for direct interaction with and binding to the antigen or epitope, i.e., eCyPA. By including these additional CDRs the affinity, specificity and selectivity and thus also prophylactic and therapeutic suitability of the antibody according to the invention and fragment thereof are thus further increased.

In embodiments, the antibody or fragment thereof according to the invention can comprise only the indicated CDR3 regions (and not the indicated CDR2 or CDR1 regions) or only the indicated CDR2 regions (and not the indicated CDR3 or CDR1 regions) or only the indicated CDR1 regions (and not the indicated CDR3 and CDR2 regions). Or, in other embodiments, the antibody or fragment thereof according to the invention can comprise the indicated CDR3 regions and preferably additionally the indicated CDR2 regions and further preferably additionally the indicated CDR1 regions.

Another embodiment of the invention provides the antibody or fragment thereof which comprises
- the heavy chain variable domains
   - CDR3 region having an amino acid sequence being at least 90% identical to said in SEQ ID NO: 1 and/or
   - CDR2 region having an amino acid sequence being at least 90% identical to said in SEQ ID NO: 3 and/or
   - CDR1 region having an amino acid sequence being at least 90% identical to said in SEQ ID NO: 5 and/or
- the light chain variable domains
   - CDR3 region having an amino acid sequence being at least 90% identical to said in SEQ ID NO: 2 and/or
   - CDR2 region having an amino acid sequence being at least 90% identical to said in SEQ ID NO: 4 and/or
   - CDR1 region having an amino acid sequence being at least 90% identical to said in SEQ ID NO: 6.

With this measure, the antigen-binding sites responsible for antigen binding and therapeutic efficacy of the particular affine and specific antibody identified by the inventors are made available. Thus, the antibody or fragment thereof according to the invention can be readily produced in a known manner.

In another embodiment of the invention said antibody or fragment thereof comprises
- a heavy chain having an amino acid sequence being at least 90% identical to said in SEQ ID NO: 7, and/or
- a light chain having an amino acid sequence being at least 90% identical to said in SEQ ID NO: 8.

In this embodiment, the entire amino acid sequences of the hypervariable regions of the heavy (IgH) and light chains (IgK) of the antibody of the invention are provided, i.e., not only the sequences of CDR1, CDR2, and CDR3, but also the sequences of the intervening 'framework regions' (FRs) FR1, FR2, FR3, and FR4. The production is thus considerably simplified and the resulting antibody and fragment are characterized by particular suitability according to the invention for the prophylaxis and treatment of a systemic disease, preferably an inflammatory systemic disease, further preferably a cardiovascular disease.

In an embodiment of the invention said antibody or fragment thereof specifically binds to human eCyPA.

The inventors were able to show that the antibody of the invention as well as fragments thereof exhibit strong and specific binding to both the human variant of eCyPA. This is particularly advantageous for prophylactic and therapeutic use of the antibody in humans.

Since no anti-eCyPA antibody is yet available in the prior art which is suitable prophylactically and/or therapeutically for use in medicine, especially in the treatment of a systemic disease, i.e., one that affects a number of organs and tissues, or affects the body as a whole, preferably an inflammatory systemic disease, further preferably a cardiovascular disease, a further object of the invention relates precisely to such an antibody or fragment thereof configured for a specific binding to eCyPA. Preferably, this antibody or fragment thereof is the antibody or fragment thereof according to the invention, which is described in detail above.

The features, characteristics, advantages and embodiments mentioned further above apply *mutatis mutandis* also for this subject-matter.

As used herein, "prophylaxis" describes the totality of all measures taken for this purpose to prevent impairment of health by risk factors, diseases or accidents. The prevention of secondary diseases or maldevelopments by timely treatment of a primary disease is also a form of prophylaxis. In relation to the invention is thought, in particular, to the prevention of the development of a systemic disease, preferably an inflammatory systemic disease, further preferably a cardiovascular disease.

According to the invention "treatment", as used in this context, refers to therapeutic measures aimed either at eliminating the cause of the disease (causal therapy) or at eliminating the symptoms (symptomatic therapy). The invention, in particular, refers to the treatment of a systemic disease, preferably an inflammatory systemic disease, further preferably a cardiovascular disease.

Still another subject-matter of the invention relates to a pharmaceutical composition characterized in comprising an antibody of fragment thereof according to the invention.

The features, characteristics, advantages and embodiments mentioned in relation with the antibody or fragment thereof apply *mutatis mutandis* also for the pharmaceutical composition.

The pharmaceutical composition may comprise a pharmaceutical carrier. As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion). The pharmaceutical composition is preferably lyophilized.

A pharmaceutical composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

These pharmaceutical compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Actual dosage levels of the active ingredient, i.e., antibody or fragment thereof, in the pharmaceutical composition of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

In an embodiment the antibody and/or fragment thereof are the only active ingredients of the pharmaceutical composition. In another embodiment, the pharmaceutical composition may contain additional active agents, such as anti-inflammatory compounds.

A still further subject-matter according to the invention relates to a kit comprising:
a) a container comprising the antibody or fragment thereof according to the invention in solution or in lyophilized formulation;
b) optionally, a second container containing a diluent or reconstituting solution for the lyophilized formulation;
c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container.

The kit of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments mentioned in relation with the antibody or fragment thereof apply *mutatis mutandis* also to the kit.

Still another subject-matter of the invention relates to a nucleic acid encoding an antibody or fragment thereof according to the invention.

This measure allows the recombinant production of the antibody or fragment thereof, according to the invention, in an advantageous manner. Methods for recombinant production are widely known in the state of the art as has been described further above.

The features, characteristics, advantages and embodiments mentioned in relation with the antibody or fragment thereof apply mutatis mutandis also for the nucleic acid.

The terms "nucleic acid" or "nucleic acid molecule", as used herein interchangeably, are intended to include DNA molecules and RNA molecules. A nucleic acid molecule may be single-stranded or double-stranded, but preferably is double-stranded DNA.

Yet, another subject-matter of the present invention is a vector, preferably an expression vector, comprising the nucleic acid according to the invention, and, optionally, regulatory elements necessary for expression in a prokaryotic and/or eukaryotic cell.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product. "Regulatory elements" of expression vectors are well known to the skilled artisan and include, e.g., promotors, enhancers, polyadenylation signals and transcription termination sequence, etc.

The features, characteristics, advantages and embodiments mentioned in relation with the antibody or fragment thereof apply *mutatis mutandis* also for the vector.

Still another subject-matter of the invention is a prokaryotic or eukaryotic host cell comprising the vector according to the invention.

The term "host cell" as used in the current application denotes any kind of cellular system which can be engineered to generate the antibodies according to the current invention. "Host cell" herein typically refers to a cell or non-human organism genetically engineered to produce the anti-eCyPA antibodies or fragments thereof according to the invention. These hosts and host cells include mammalian cells, bacterial, yeast, insect cells, plant cells and transgenic plants or animals such as rodents, plants and bovines. Typically, antibodies or antibody fragments are expressed in mammalian, bacterial and yeast cells. In one embodiment HEK293 cells and CHO cells are used as host cells.

The features, characteristics, advantages and embodiments mentioned in relation with the antibody or fragment thereof apply *mutatis mutandis* also for the prokaryotic or eukaryotic host cell.

Another subject-matter of the invention relates to a method for the prophylaxis and/or treatment of a systemic disease, preferably an inflammatory systemic disease, further preferably a cardiovascular disease, in a living being, comprising the administration of a prophylactically and/or therapeutically effective amount of the antibody or fragment thereof and/or the pharmaceutical composition according to the invention.

The features, characteristics, advantages and embodiments mentioned in relation with the antibody or fragment thereof apply mutatis mutandis also for the method according to the invention.

A "living being" refers to any subject or organism, including mammals, in particular humans.

The invention is now further explained by means of embodiments and examples resulting in additional features, characteristics and advantages of the invention. The embodiments and examples are of pure illustrative nature and do not limit the scope or range of the invention. The features mentioned in the specific embodiments and examples are features of the invention and may be seen as general features which are not applicable in the specific embodiment or example but also in an isolated manner in the context of any embodiment or example of the invention.

The invention is now further described and explained in more detail by referring to the following non-limiting examples and figures.

### BRIEF DESCRIPTION OF THE FIGURES

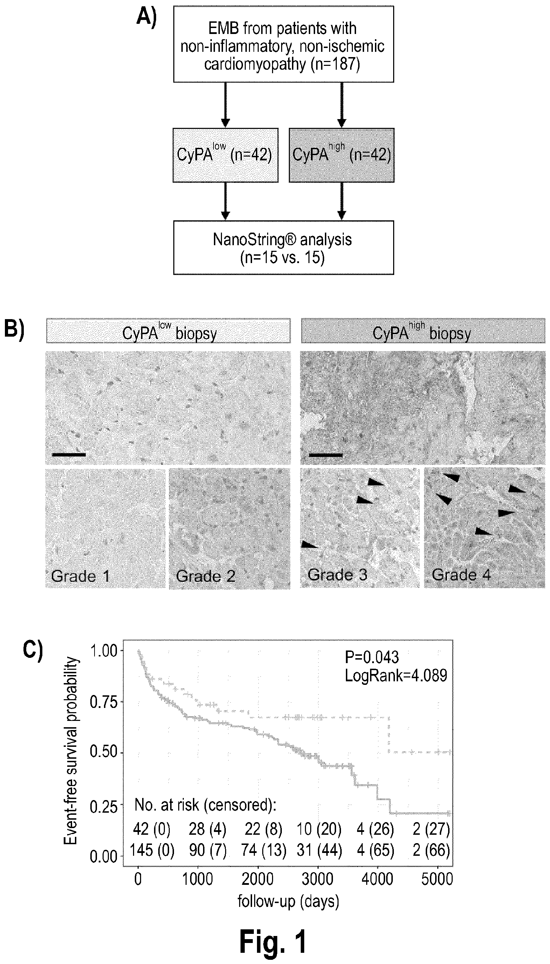

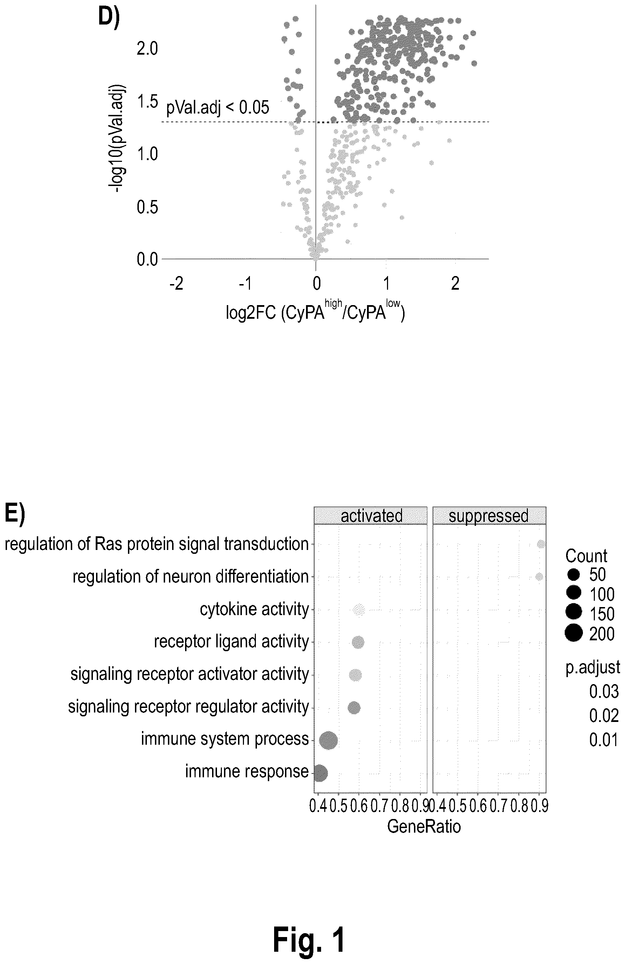

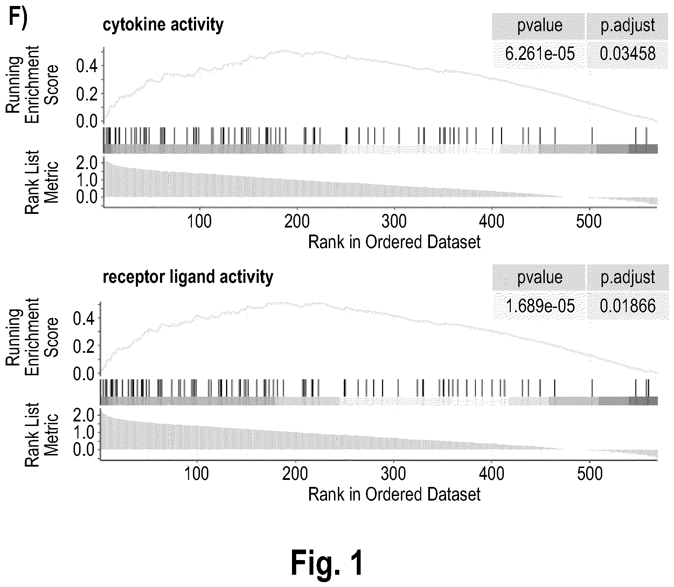

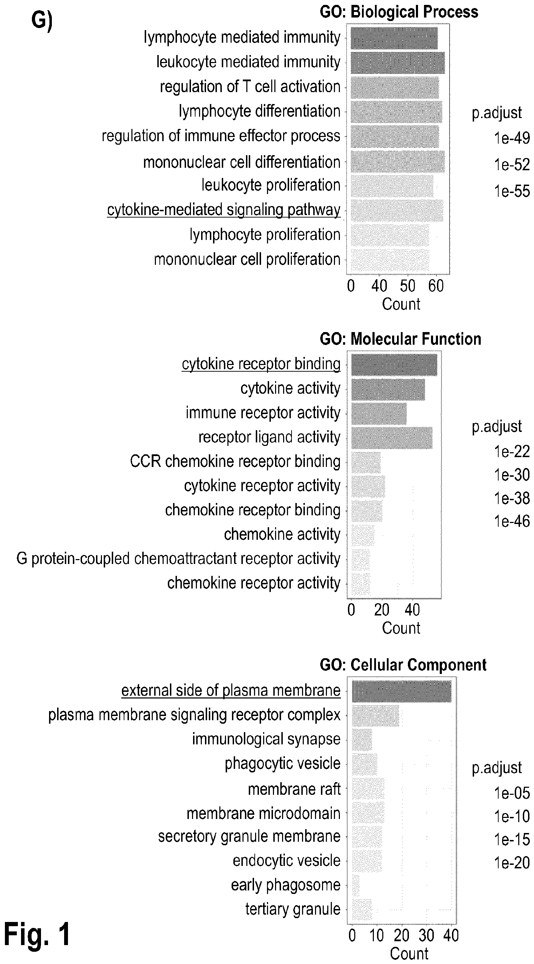

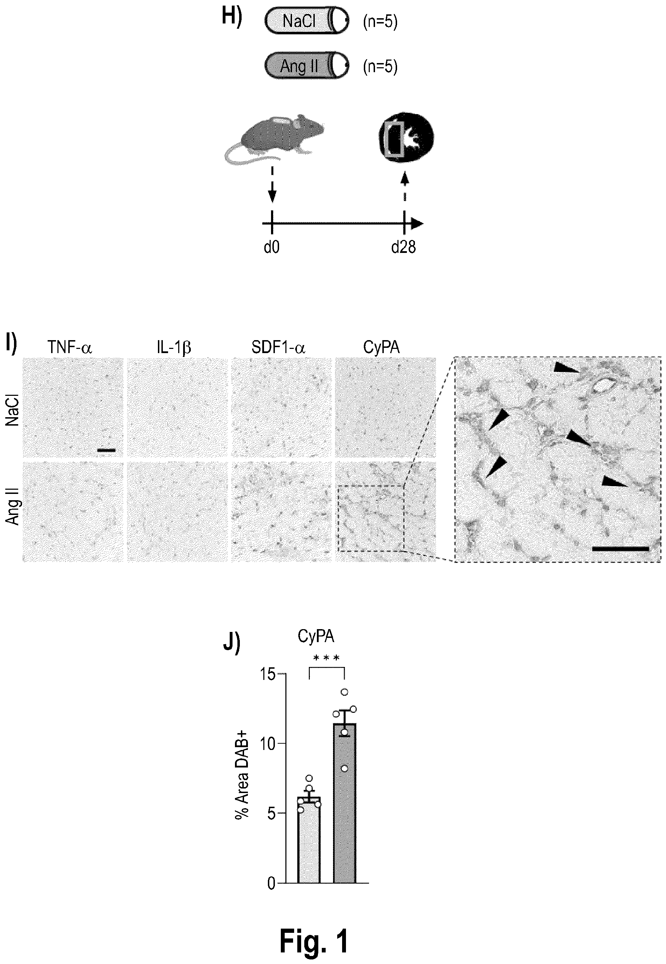

- Fig. 1: Figure 1. Accumulation of CyPA in the cardiac microenvironment is associated with poor prognosis in patients with congestive heart failure. A Endomyocardial biopsies (EMB) from 187 patients with non-inflammatory, non-ischemic cardiomyopathy were histologically assessed by immunohistochemical staining for CyPA. The cohort was then divided into a subgroup of patients with low and high expression of CyPA. From 30 of these biopsies NanoString^{®} mRNA profiling was performed. B Representative biopsies from patients with low and high CyPA expression and graduation are shown. In CyPA^{high} biopsies, CyPA was observed to accumulate in the extracellular space (black arrows). Scale bar, 50 pm. C Kaplan-Meier curves for cumulative event-free survival of patients with CyPA^{low} vs. CyPA^{high} biopsies show a clear decline in patients with a high CyPA expression pattern. Further characterization of patient cohort is provided in Supplemental Table S1. D Volcano plot from NanoString^{®} analysis shows an upregulation of mRNA transcripts of inflammatory regulators in non-inflammatory cardiomyopathy. E, F Gene Set Enrichment Analysis (GSEA) demonstrates strongly upregulated pathways of cytokine activity and signaling receptor activity. G Gene Ontology (GO) analysis in the three distinct aspects of biological process, molecular function, and cellular component imply increased biological processes at the external side of the plasma membrane, including enhanced cytokine receptor binding and cytokine-mediated signaling pathways. H *ApoE-*/*-* mice were challenged with Ang II or NaCl (saline) via osmotic mini-pumps over 28 days, to mimic non-ischemic, non-inflammatory cardiomyopathy *in vivo.* I Cytokine profiling from immunohistochemical stainings for TNF- , IL-1 , SDF-1 and CyPA. CyPA showed a clear accumulation in the extracellular space (black arrows). Scale bar. 200 µm. J Expression of CyPA was significantly enhanced in mice treated with Ang II over 28 days compared to healthy mice. Plotted: mean ± S.E.M., unpaired t-test, n = 5. EMB endomyocardial biopsy, FC fold change.

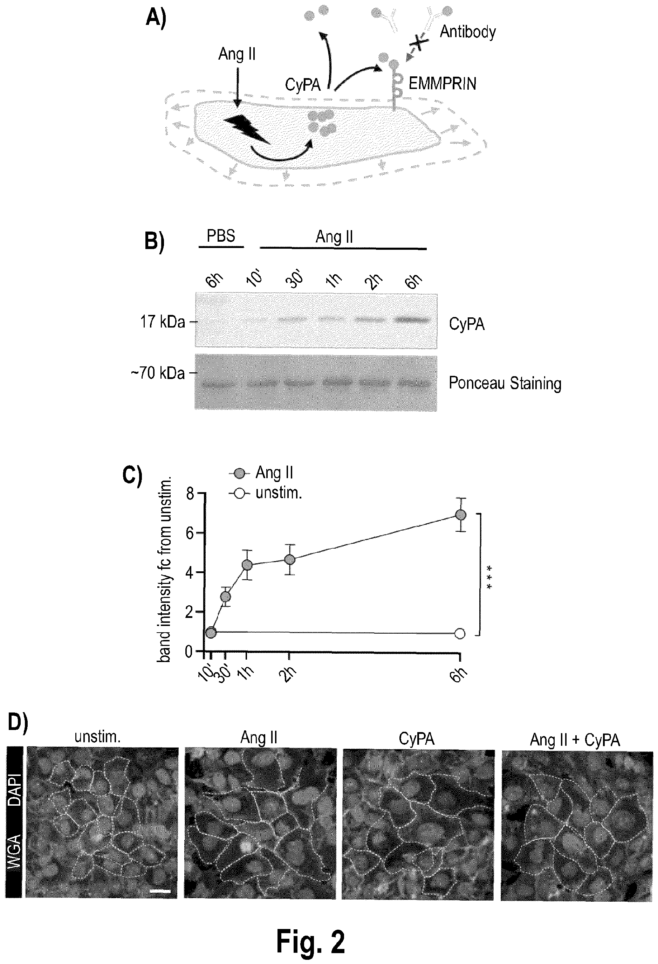

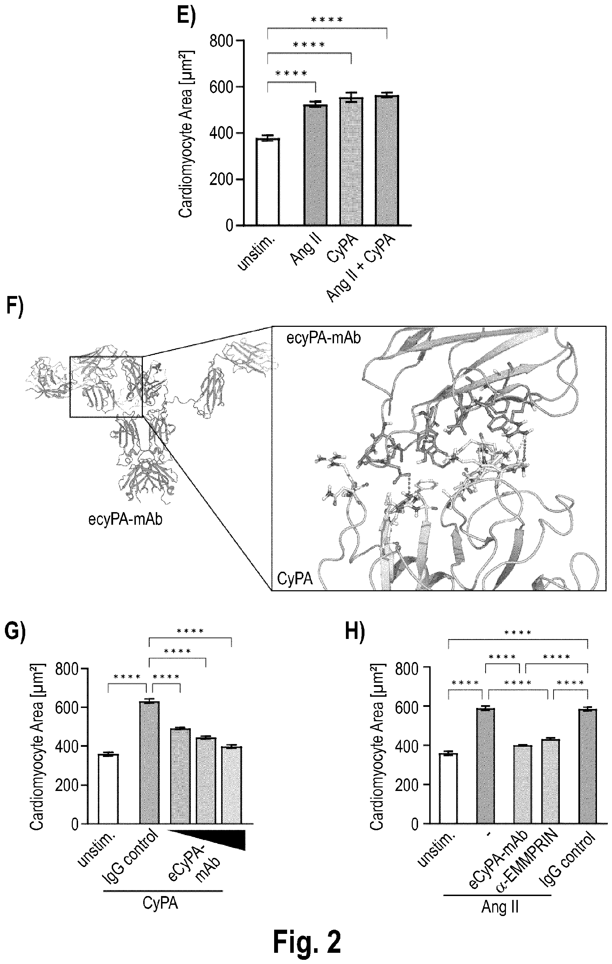

- Fig. 2: Neutralizing extracellular CyPA prevents Ang II-induced cardiomyocyte hypertrophy *in vitro.* A Graphical abstract of the hypothesis that Ang II stresses cardiomyocytes to release CyPA, which then likely acts in autocrine and paracrine fashion via its receptor EMMPRIN. Neutralizing secreted CyPA by an antibody is supposed to inhibit pathophysiological remodeling of cardiomyocytes, including cellular hypertrophy. B CyPA is released from HL-1 cardiomyocytes in a time-dependent manner, as quantified by Western Blot analysis from the medium of cells treated with Ang II or PBS (unstimulated). C Cy-PA band intensity was quantified as fold change from unstimulated cells after 6 hours. n = 4. D Chronic challenge of HL-1 cardiomyocytes over 48 hours with Ang II or CyPA promotes cellular hypertrophy, as assessed by immunofluorescence stainings of the cell membrane with fluorescently labeled wheat germ agglutinin (WGA). Scale bar, 25 um. E Cardiomyocyte was significantly enhanced after treatment with Ang II or CyPA, but both together showed no synergistical effects, n = 4. F Antibodies were generated to specifically target the EMMPRIN binding site of CyPA. Protein interaction modeling shows putative antibody-antigen complex model of the most effective antibody (in green ribbons and brownish carbons) and the interaction with CyPA (in turquoise ribbons and white carbons) using the Schrddinger Prime approach. G In vitro testing of the most promising antibody shows a dose-depending effeet on cardiomyocyte hypertrophy. n = 5. H Comparison of a commercially available, receptor- blocking antibody against EMMPRIN demonstrated comparable results on inhibiting cardiomyocyte hypertrophy. n = 5. Plotted: mean i S.E.M, one-way ANOVA with Tukey's posthoc test for multiple comparison.

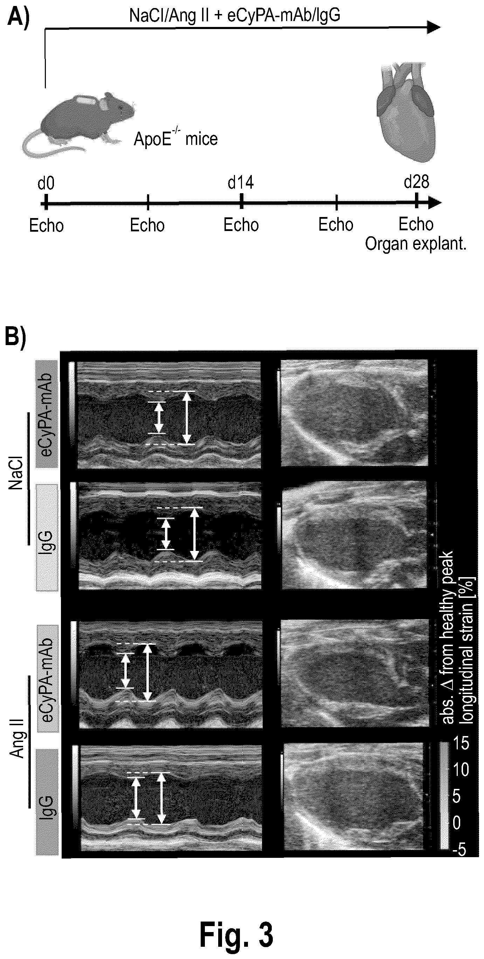

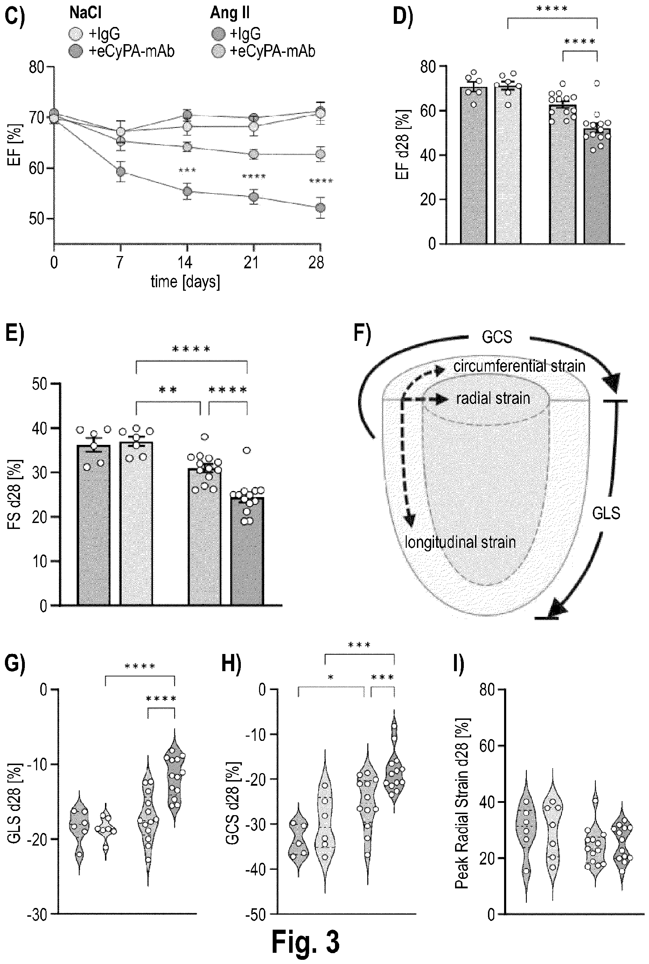

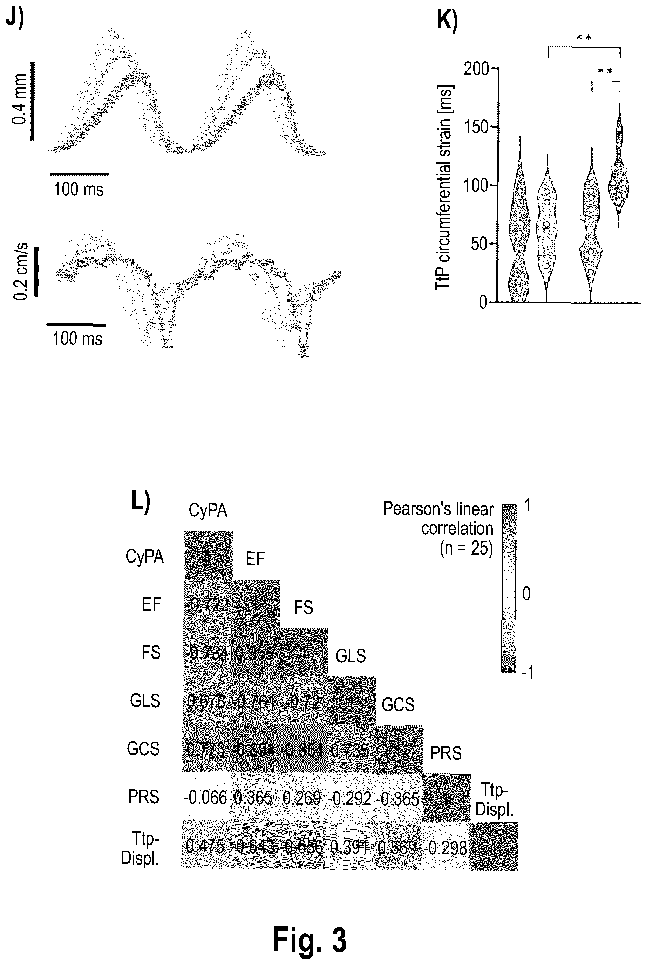

- Fig. 3: Myocardial function in Ang II-treated mice is preserved by targeting eCyPA. A *ApoE*^{*-*/*-*} mice were continuously infused with Ang II or NaCl (saline) over 28 days. The developed anti-eCyPA-antibody or its IgG isotype control were administered for both groups respectively, to control effects of systemic eCyPA inhibition. Cardiac function was examined by repeated echocardiographic measurements every 7 days. n described in Methods. B Representative echocardiographic images of the parasternal short axis in M-Mode, demonstrating a clear decline in heart contractility of mice treated with Ang II + IgG over 28 days (left). Exemplary strain overlay images display a rather apical focus of peak longitudinal strain decline. C Echocardiographic analysis of the ejection fraction over a time course of 28 days by repeated measurements. D, E After 28 days of treatment, a deterioration in heart function (EF = ejection fraction, F8 = fractional shortening) was significantly attenuated by anti-eCyPA treatment. F Cardiac contractility was assessed by comprehensive speckletracking based strain analysis. GLS global longitudinal strain, GCS global circumferential strain. G, H, I Quantification of GLS, GCS and peak radial strain. J Temporal resolution of displacement of the cardiac wall during contraction of two cardiac cycles (averaged from short axis view, one representative mouse per group). In mice treated with Ang II + IgG, the peak displacement (top) during systole is delayed, and the peak velocity of relaxation is enhanced vice versa. K Quantification of the time-to-peak (TtP) for circumferential strain. L Correlation plot for Spearman's pair-wise linear correlation between CyPA expression measured by immunohistochemical stainings and cardiac function measured by echocardiography. n = 25. Plotted: mean i S.E.M (barplots) or mean and quartiles (dashed, violin plots). Two-sided ANOVA with Tukey's posthoc test for multiple comparisons. PRS Peak radial strain.

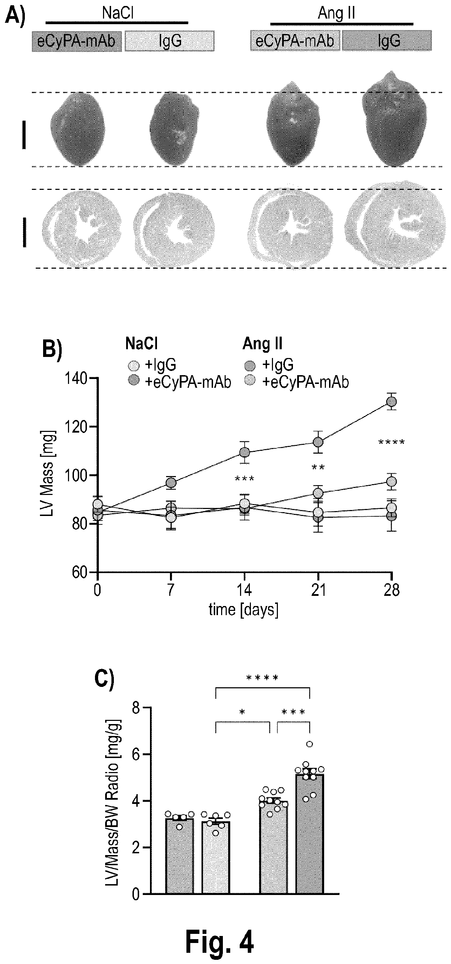

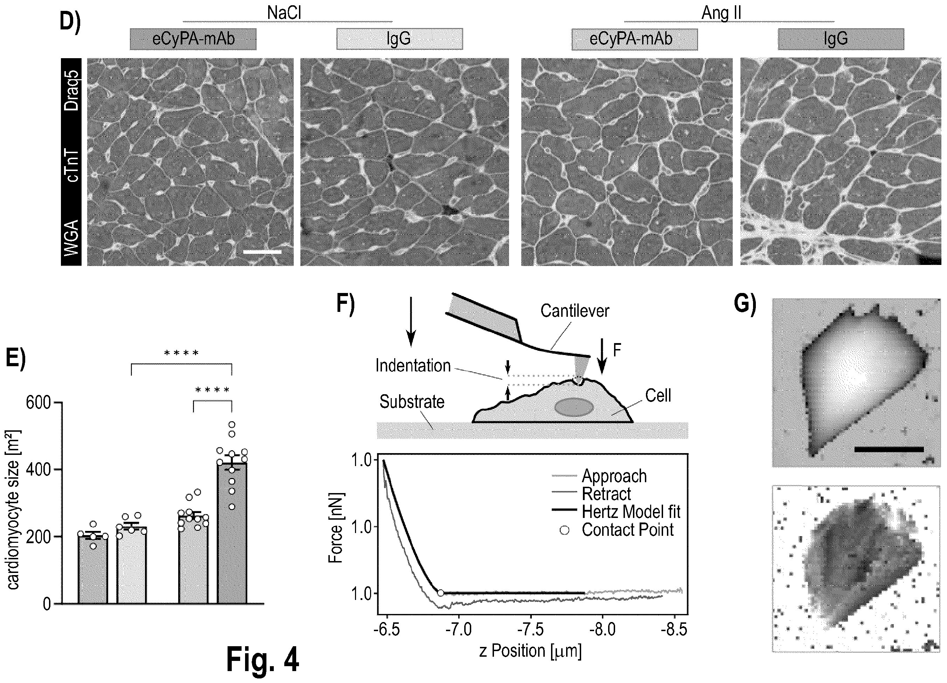

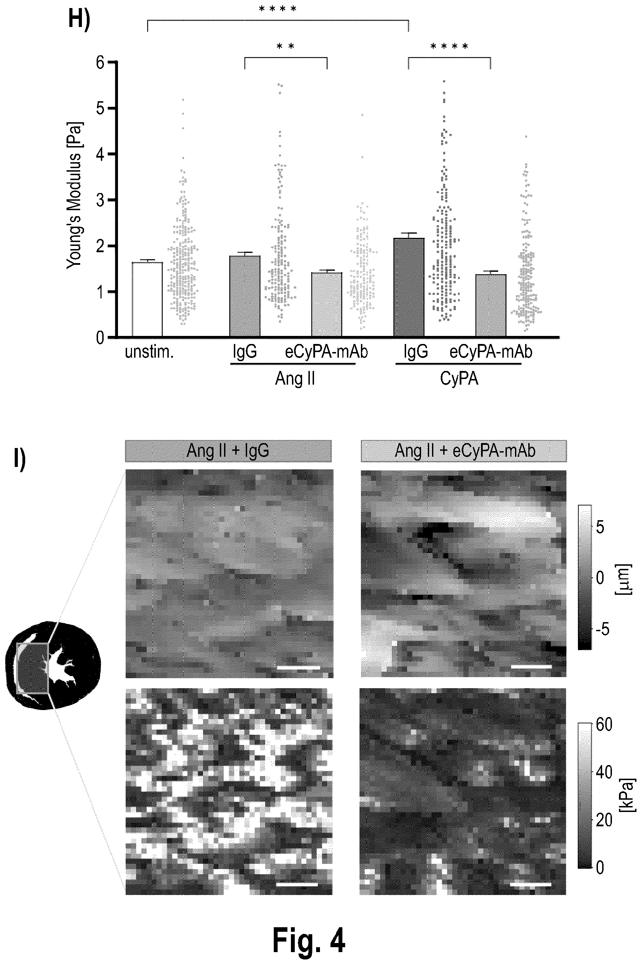

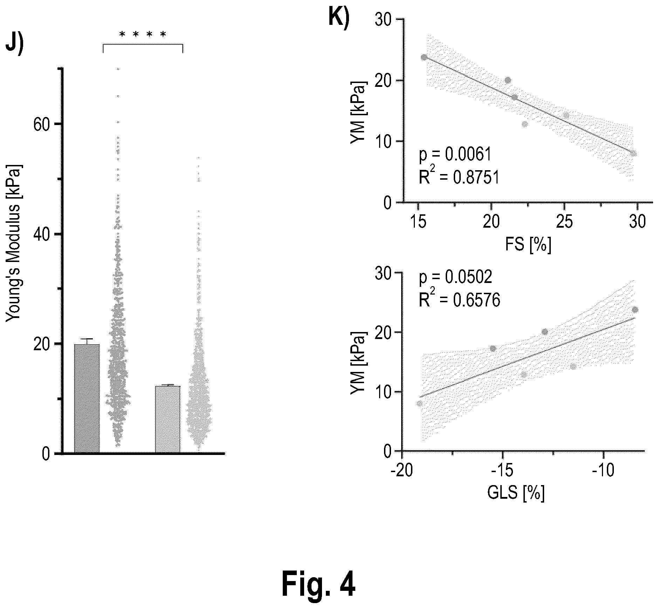

- Fig. 4: Myocardial hypertrophy and stiffening are promoted by eCyPA *in vitro* and *inlex vivo.* A Representative *ex vivo* photographs of explanted hearts and H&E staining of hearts at the short axis center. Scale bar, top 5 mm, bottom 3 mm. B Time course of left ventricular (LV) mass, measured every 7 days by echocardiography. n described in Methods. C Quantification of the ratio between LV mass determined by echocardiography and body weight after 28 days of treatment. D Representative immunofluorescence stainings of heart sections from the heart septum. Cardiomyocyte size was assessed by staining of the cell membrane with fluorescently labeled wheat germ agglutinin (WGA, green), Troponin T (cTnT, red), and nuclei (Draq5, blue). Scale bar, 20 µm. E *Ex vivo* quantification of cardiac hypertrophy by cardiomyocyte sizes. F Schematic of atomic force microscopy (AFM) measurements for *in vitro* and ex *vivo* cell stiffness. The force for indentation of the cell surface by a cantilever is measured and the Hertz model is then fitted to the force curve to (YM, stiffness). G Representative images for a single HL-1 cardiomyocyte: surface topography (top) and YM (bottom). Scale bar, 20 µm. H Quantification of HL-1 YM after 48 hours of treatment with Ang II or CyPA, each together with eCy-PA-mAb or IgG control. One-way ANOVA . n = 5. I Representative AFM topography (top) and YM (bottom) images of 30 µm thick sections of Ang II-treated mice from the *in vivo* experiment. Scale bar, 10 µm. J YM at the inter-ventricular septum of each section was measured and compared between eCyPA-mAb or IgG control treated mice. U t-test. n = 3. K *In vivo* stiffness measured by echocardiography shows strong correlation to *ex vivo* stiffness assessed by AFM. Plotted: mean ± S.E.M. LV left ventricular, BW body weight, YM Young's modulus.

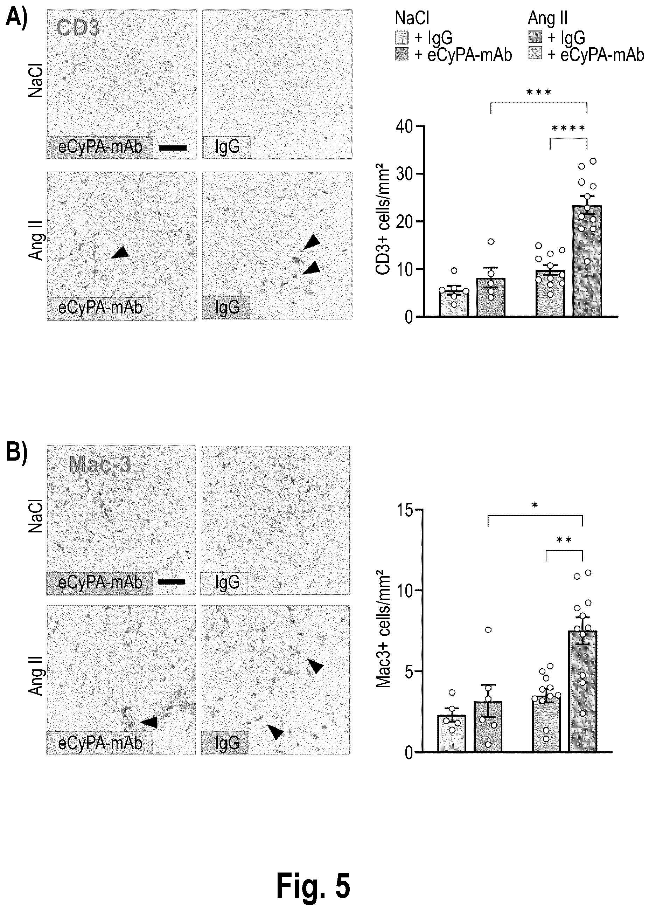

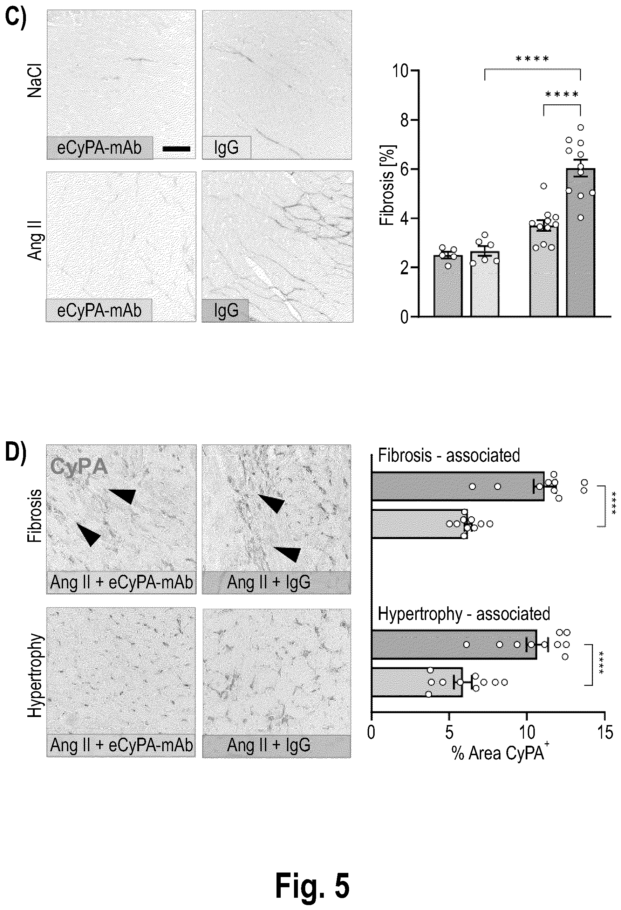

- Fig. 5: Inhibition of eCyPA attenuates Ang II-induced myocardial inflammation and fibrosis. A, B Inflammation was investigated by immunohistochemical stainings of CD3⁺ T cells (A) and Mac-3⁺ macrophages (B). The number of cells was counted for the whole section. Scale bar, 20 µm. C Representative images of Picro-Sirius Red stainings, which were used to quantify fibrosis for the whole section. Scale bar, 100 pm. D CyPA expression was studied by immunohistochemistry and showed accumulation predominately in areas previously identified as fibrotic or hypertrophied. Plotted: mean i S.E.M. Two-sided ANOVA with Tukey's posthoc test for multiple comparisons. n described in Methods.

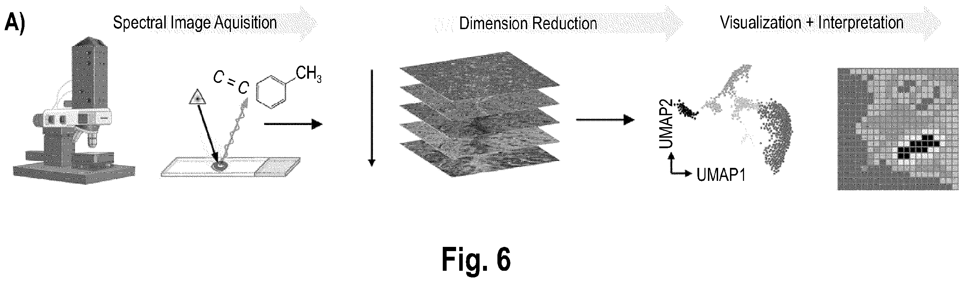

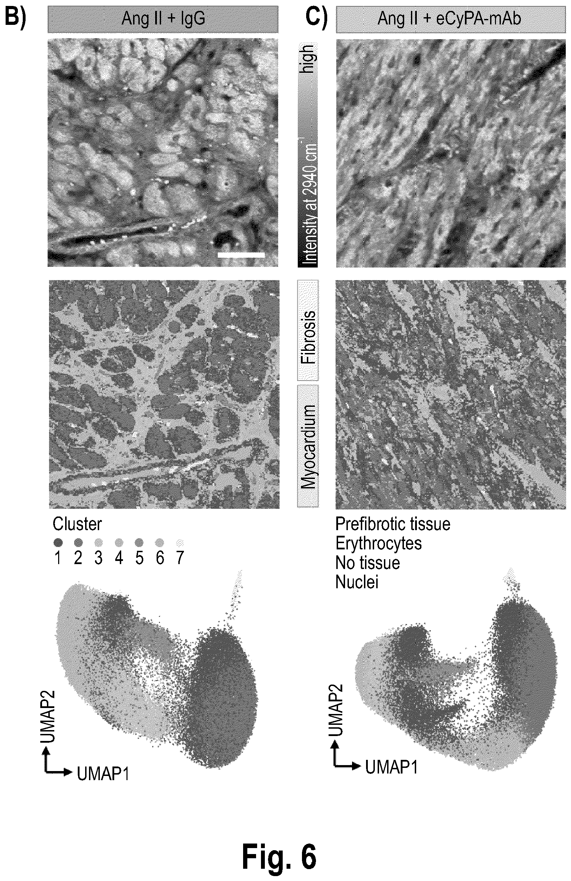

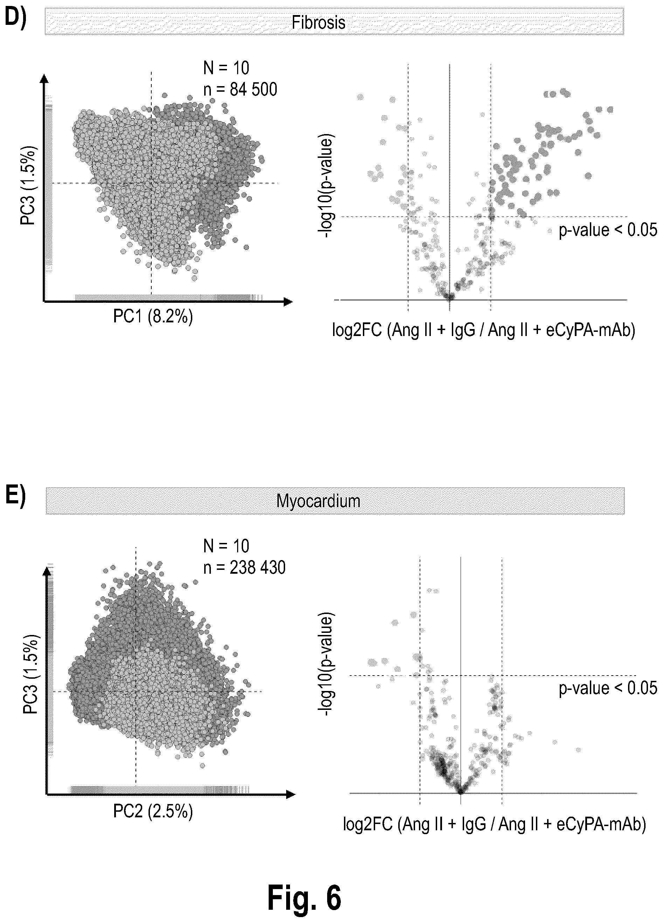

- Fig. 6: Raman spectroscopy deciphers molecular rearrangements in myocardium and fibrosis of failing hearts. A Schematic outline for the analytical workflow of Raman "Spectromics" of cardiac matrix. Briefly, regions of interest were scanned with a resolution of 1 pm using a Raman microspectroscope. Untargeted spectra were loaded into the Seurat workflow (Methods) to obtain a clustering of similar spectra and their spatial representation. Identified meaningful clusters where then analyzed across experimental conditions. B, C Representative comparison between the two experimental groups and the Raman scan area at a general protein band (2940 cm⁻¹) (top), generated cluster image (middle) from unsupervised clustering, as also shown in UMAP projection (bottom). Scale bar, 50 µm. D Principal component analysis (PCA) of spectra from n=5 mice per group shows a clear separation of fibrosis-related spectra into two partially overlapping clusters. Volcano Plot (right) visualization highlights highly significant and strong changes of specific Raman peaks. E PCA (left) and Volcano Plot (right) for spectra related to myocardium. N individual mice, n cumulative spectra. Log2FC log-transformed foldchange of intensities found for Ang II + IgG relative to Ang II + eCyPA-mAb.

### EXAMPLES

### 1. Introduction

While previous approaches have shown significant effects on cardiac hypertrophy by global CyPA deficiency, the inventors' study aimed to specifically investigate the pathomechanistic aspects of secreted, extracellular CyPA (eCyPA) and its contribution to myocardial stiffening and dysfunction in the context of cardiac hypertrophy. The inventors hypothesized that the secreted form of CyPA could exert detrimental effects on cardiac function through its interactions with the ECM and signaling pathways. To test this hypothesis, the inventors employed a combination of cutting-edge *in vitro* and *in vivo* approaches with both murine and human tissue samples. The results of the inventors revealed a significant accumulation of eCyPA in cardiac hypertrophy, suggesting its potential involvement in the pathogenesis of this condition. Importantly, the inventors observed a strong correlation between eCyPA expression and the severity of myocardial stiffening and dysfunction. Furthermore, the inventors investigated the effects of inhibiting the extracellular accumulation of CyPA by a specific monoclonal antibody and found that this intervention effectively attenuated the pathological consequences of eCyPA.

The findings of the inventors highlight the pathogenic role of extracellular CyPA in myocardial stiffening and dysfunction associated with cardiac hypertrophy and remodeling. Moreover, the inventors suggest that therapeutic strategies targeting the extracellular accumulation of CyPA may hold promise for the treatment of adverse cardiac remodeling in non-ischemic congestive heart failure.

### 2. Methods

### Data Availability

The datasets generated or analyzed for the current study are available from the corresponding author upon reasonable request and authorization of the local ethics committee.

### Antibody generation

For generation of antibody candidates targeting the EMMPRIN-binding site of CyPA, rats were immunized with a peptide containing the lead sequence of TAKTE which was identified as EMMPRIN binding site previously. In detail, immunization was performed by a subcutaneous injection of a mixture of 50 µg ovalbumin-coupled lead sequence (OVA, Peps4LS, Heidelberg, Germany), 5 nmol/L CPG oligonucleotides (Tib Molbiol, Berlin, Germany), 500 µl PBS and 500 µl incomplete Freund's adjuvant. Antibodies were then generated fusing spleen cells with immortal myeloma cells, in order to create monoclonal hybridoma cell lines that express the specific antibodies. Purified antibodies were then tested *in vitro* and the most promising candidate was submitted to further testing. For in silico modeling, the inventors generated a putative antibody-antigen complex model after sequencing of the most promising antibody candidate. The antibody was modelled using the Schrödinger Prime approach while the antigen was based on PDB-ID 7ABT. The complex structure was created by Schrödinger Protein-Protein docking module.

### Antibody variable domain sequencing

Total RNA was isolated from the hybridoma cells following the manufacturer's instructions. Total RNA was then reverse-transcribed into cDNA using either isotype-specific anti-sense primers or universal primers following the technical manual of SMARTScribe Reverse Transcriptase. Antibody fragments of heavy chain and light chain were amplified according to the standard operating procedure (SOP) of rapid amplification of cDNA ends (RACE) of GenScript. Amplified antibody fragments were cloned into a standard cloning vector separately. Colony PCR was performed to screen for clones with inserts of correct sizes. The consensus sequence was provided.

### Human Studies

A total of 187 patients underwent endomyocardial biopsy at the inventors' department as part of an evaluation for non-ischemic congestive heart failure between 2004 and 2011. Indications for endomyocardial biopsy were based on the s initial individual clinical presentation and of individual decision of the respective treating cardiologist according to current guidelines. All patients received medication according to current European Society of Cardiology (ESC) and American College of Cardiologists (ACC)/American Heart Association (AHA) guidelines depending on their left ventricular function and heart failure symptoms. After rule out of coronary artery disease by coronary angiography, endomyocardial biopsies were extracted from the interventricular septum and fixed in 4% buffered formaldehyde. Biopsies were evaluated for their relative CyPA expressions by laboratory technicians blinded to patient data and experimental conditions. The study was approved by the local ethics committee (Project-No.253/2009BO2) and complies with the declaration of Helsiniki and the good clinical practice guidelines.

### Animal Experiments

Osmotic mini-pumps (model 1004, Alzet, Durect Corporation, Cupertino, CA, USA) were subcutaneously implanted into 8-12 weeks old male ApoE^{-/-} mice (Charles River, Massachusetts, USA). The pumps delivered Angiotensin II (Cat. A9525, Sigma-Aldrich, St. Louis, Missouri, USA) at a continuous delivery rate of 1000 ng/min per kg body weight or NaCl 0,9% (B. Braun, Melsungen, Germany) at a flow rate of 0.11 µl per hour, in combination with a body weight-adapted dose of the eCyPA-mAb or its rat IgG control. Mice were sacrificed after 28 days of pressure-overload injury, resulting in cardiac hypertrophy and remodeling. Transthoracic echocardiography was performed on day 0 immediately before implantation of the osmotic pumps, and on days 7, 14, 21 and 28. All echocardiographic studies were conducted on a Vevo2100 imaging station (FUJIFILM VisualSonics Inc., Toronto, Ontario, Canada). Data analysis was performed using VisualSonics VevoLab 3.0.5 and MATLAB (MathWorks Inc., Natick, MA, USA) as described previously. All animal procedures were performed according to the German animal protection law and approved by the local authorities (Regierungspräsidium Tübingen, TVA M04/19G and 184 M02/20G).

### Tissue-based comprehensive phenotyping

Paraffin (FFPE) or cryosections were generated as described in more detail in the Supplemental Material and Methods. For Nanostring^{®} nCounter Analysis System assay (NanoString^{®} Technologies, Seatlle, USA), total RNA was extracted from FFPE sections using the RNeasy FFPE Kit (Qiagen, and as described previously. mRNA expression was then measured with the NanoString nCounter assay with 100 ng of total RNA. Expression data were analyzed utilizing the NanoString nSolver Analysis Software v3.0, considering positive and negative control, housekeeping and total (excluding controls) counts as well as the binding densities in each sample.

For *ex vivo* stiffness measurements, 30 µm cryosections from hearts of mice after 28 days of treatment were generated and mounted on Superfrost^{®} Plus microscope slides (R. Langenbrinck GmbH, Emmendingen, Germany) as described previously. Briefly, cross-sections were thawed and measured in PBS (Dulbecco's Phospahte Buffered Saline, Sigma-Aldrich, Taufkirchen, Germany). Atomic force microscopy was performed with a home-built setup using sphere tip cantilevers (biosphere B1000-CONT, Nanotools GmbH, München, Germany). The spring constant of cantilevers was calibrated using the thermal noise method. Data were analyzed using IGOR PRO 9 (WaveMetrics, Inc., Portland, USA). The Young's modulus was obtained from the measures force curves by fitting the Hertz model.

For Raman spectroscopy, FFPE sections were scanned at 1 µm resolution on a Witec alpha 300R confocal Raman microscope (Witec GmbH, Ulm, Germany) coupled with a green laser (532 nm) at 50 mW laser power, an integration time of 0.05 s/pixel and at 63x magnification in an area of 250x250 µm. Raw spectral data from Raman scans were preprocessed using Project FIVE 5.2 (Witec, Ulm, Germany), as described previously and then integrated into the Seurat workflow for unsupervised machine learning cluster analysis. Cluster analysis helped separating the scan in various myocardial compartments based on similar spectral signals. Fibrotic and myocardial spectral signatures were then compared between Ang II-infused mice treated with anti-eCyPA and IgG control.

### Statistical Analysis

Statistical analysis was performed with RStudio running R 4.2.3., GraphPad Prism 1.9.2 (GraphPad Software, La Jolla, California, USA), and IBM SPSS Statistics software version 26 (SPSS Inc., Chicago, Illinois, USA). Basic charts (bar plots, violin plots) were created with GraphPad, while more sophisticated figures were made with R. Data are presented as mean ± S.E.M, as indicated for each figure. Data was tested for normal distribution by Shapiro-Wilk normality test and significance was then calculated using one-way or two-way ANOVA with Tukey t-test, as indicated in each figure legend. Statistical significance level was defined at 0.05. All experiments were performed and analyzed in a blinded fashion.

### 3. Results

### CyPA accumulates in the myocardial microenvironment in chronic heart failure

CyPA is an independent predictor of clinical outcome in patients with congestive heart failure, as the inventors have shown in a previous study. While CyPA is predominantly secreted in inflammatory settings, the inventors studied CyPA expression in endomyocardial biopsies taken from patients (n=187) with non-inflammatory, non-ischemic congestive heart failure (Figure 1A). Within this cohort, the inventors ranked CyPA expression along an intensity score to divide the cohort into a CyPA^{low} and CyA^{high} expression subgroup. Myocardial CyPA expression was substantially enhanced in 78% (n=145) of the cohort (Figure 1A). Interestingly, enhanced myocardial expression of CyPA was found not only intracellularly but also in the extracellular space of the myocardium (Figure 1B). For the two subgroups, CyPA expression indicated a critical impact on adverse clinical events, including death, survived sudden cardiac death (ICD shock delivered for VF or VT), heart transplantation, and re-hospitalization due to heart failure with need for intensive care treatment. Patients with high CyPA expression (CyPAhigh) had a significantly higher event rate in comparison to patients with low expression (CyPAlow) over a follow-up period of nearly 15 years (5195 days, P=0.043, LogRank = 4.089) (Figure 1C).

To further characterize the underlying inflammatory pathways in these failing human hearts, the inventors performed NanoString^{®} mRNA profiling from the endomyocardial biopsies using a custom panel of 594 inflammation-related genes. Volcano plot and heatmap analysis demonstrated a significant overall upregulation of inflammatory mediators in CyPAhigh compared to CyPAlow samples (Figure 1D), although only patients with non-inflammatory cardiomyopathy were enrolled. Gene Set Enrichment Analysis (GSEA) implied a strong upregulation of cytokine production, cytokine activity, cytokine receptor binding and receptor-ligand activity (Figure 1E, F). The most prominently downregulated transcripts comprised the regulation of Ras protein signaling (Figure 1E). A deeper look into the Gene Ontology (GO) aspect of biological process suggested strong involvement of lymphocyte and leukocyte-mediated immunity as well as a pronounced upregulation of cytokine-mediated signaling pathways, supporting the influence of sterile myocardial inflammation even in non-inflammatory, non-ischemic cardiomyopathy. Strikingly, the GO aspect of cellular component and molecular function suggested upregulation of cytokine receptor binding, and accordingly, the external side of plasma membrane and secretory granule membranes were identified as location of these processes (Figure 1E). These results go hand in hand with the inventors' previous histological finding of extracellular accumulation of CyPA.

To further investigate myocardial CyPA expression and its pathophysiological implications the inventors used a robust murine model of chronic heart damage, by continuously infusing Angiotensin II (Ang II) into atherosclerosis-prone apolipoprotein E (ApoE)-deficient mice over 28 days (Figure 1H). Immunohistochemical analysis of different cytokines (TNF- -CXCL12/SDF- CyPA) showed a significantly enhanced protein expression of CyPA in Ang II-treated mice compared to control mice (P=0.0009) (Figure 1I, J). Most strikingly, CyPA was primarily found in the extracellular microenvironment, replicating the previously described pattern found in human biopsies (Figure 1I, right). Thus, in both human and murine hearts, myocardial CyPA accumulation in the extracellular microenvironment is associated with congestive heart failure. The inventors hypothesized that CyPA is secreted into the extracellular space in response to chronic heart damage and here critically interacts with its receptor to accomplish a presumably pathophysiological remodeling.

### Antibody-based inhibition of extracellular CyPA prevents Ang II-induced cardiomyocyte hypertrophy in vitro

To further study the mechanistic role of eCyPA in the process of failing hearts, the inventors studied CyPA release and its functional implications in vitro using an immortalized murine cardiomyocyte cell line (HL-1). The inventors postulated that CyPA is secreted into the pericellular space upon stimulation with Ang II, comparable to a danger-associated molecular pattern (DAMP). From its extracellular location, CyPA may bind to the surface of adjacent cells or the same cell, acting in a paracrine or autocrine manner (Figure 2A). The most prominent CyPA receptor is EMMPRIN, which promotes cell hypertrophy via ERK1/2 signaling, as shown previously.

Upon stimulation with Ang II, CyPA is released from cultured cardiomyocytes in a time dependent manner (P<0.001) (Figure 2B, C). To study chronic effects of cardiomyocyte stimulation, HL-1 cells were cultivated in the presence or absence of Ang II (400 nM), CyPA (200 nM) or the combination of both for 48 hours, and cellular hypertrophy was quantified by staining the cell membrane and measuring the cell size (Figure 2D, E). When HL-1 cells were treated with Ang II, cell size was significantly increased compared to untreated cells. Similar effects on cell size were observed in the presence of extracellular CyPA, indicating that both Ang II and CyPA induce hypertrophy of cardiomyocytes. No synergistic effect on cell size was observed when both mediators were present (Figure 2E). The inventors complemented the previous *in vitro* findings by a conditioned medium assay: In order to elucidate the paracrine effect of CyPA on cell hypertrophy, the conditioned medium of Ang II- or PBS-stimulated HL-1 cells was transferred to untreated cells and changes of cell size were evaluated in the presence of anti-CyPA or IgG control. As observed before, eCyPA-mAb significantly prevented HL-1 cells from Ang II-supernatant-induced cell size enlargement compared to IgG control.

To break down CyPA-mediated effects to its extracellular localization, the inventors aimed to neutralize secreted CyPA. However, most of the currently available CyPA inhibitors block both the intra- and extracellular function of CyPA. In order to specifically address the role of e CyPA on myocardial hypertrophy and heart failure, the inventors developed a neutralizing CyPA monoclonal antibody (mAb) directed against the active EMMPRIN receptor binding site of CyPA, and therefore restricting effects to only the extracellular function. The antibody was generated by using the lead peptide TAKTE as immunogen, targeting the identified EMMPRIN epitope of Cyclophilin A. Various candidates were screened *in vitro* for their capacity to inhibit cardiomyocyte hypertrophy upon Ang II stimulation. The most promising antibody was subjected to sequence analyses of immunoglobulin variable region.

Part of the application and disclosure is a sequence listing in WIPO St.26-Forrmat. In the following, all sequences are again listed which are subject invention. In the event of discrepancies between the sequences in the attached sequence listing and the sequences listed in the following, the sequences listed in the following shall take precedence.

### Amino acids

*Heavy chain CDR3 amino acid*
   HFATVESY (SEQ ID NO: 1)
*Light chain CDR3 amino acid*
   MQATHAPWT (SEQ ID NO: 2)
*Heavy chain CDR2 amino acid*
   YITRSSDTVYADAVKG (SEQ ID NO: 3)
*Light chain CDR2 amino acid*
   LVSNLGS (SEQ ID NO: 4)
*Heavy chain CDR1 amino acid*
   RYGMH (SEQ ID NO: 5)
*Light chain CDR1 amino acid*
   RSSQSLLDSDGNTYLY (SEQ ID NO: 6)
*Heavy chain full amino acid* (*signal peptide-FR1-CDR1-FR2-CDR2-FR3-CDR3-FR4)* 
MYFSISLFFLVLILKSVQCEVQLVESGGGLVQPGKSLKLSCSASGFTFSRYGMHWIRQAP
GKGLDWVAYITRSSDTVYADAVKGRFTISRDNAKSTLYLQLNSLKSEDTAIYYCASHFATV
ESYWGQGVMVTVSS (SEQ ID NO: 7)
*Light chain full amino acid (signal peptide-FR1-CDR1-FR2-CDR2-FR3-CDR3-FR4)* 
MSPVQSLFLLLLWILGTKGDVVLTQTPSILSVTVGQSVSISCRSSQSLLDSDGNTYLYWFL
QRPGQSPQRLIYLVSNLGSGAPNRFSGSGSGTDFTLKISGVEAEDLGVYYCMQATHAP
WTFGGGTKLELK (SEQ ID NO: 8)
*Heavy chain FR1*
   EVQLVESGGGLVQPGKSLKLSCSASGFTFS (SEQ ID NO: 9)
*Heavy chain FR2*
   WIRQAPGKGLDWVA (SEQ ID NO: 10)
*Heavy chain FR3*
   RFTISRDNAKSTLYLQLNSLKSEDTAIYYCAS (SEQ ID NO: 11)
*Heavy chain FR4*
   WGQGVMVTVSS (SEQ ID NO: 12)
*Light chain FR1*
   DWLTQTPSILSVTVGQSVSISC (SEQ ID NO: 13)
*Light chain FR2*
   WFLQRPGQSPQRLIY (SEQ ID NO: 14)
*Light chain FR3*
   GAPNRFSGSGSGTDFTLKISGVEAEDLGVYYC (SEQ ID NO: 15)
*Light chain FR4*
   FGGGTKLELK (SEQ ID NO: 16)

### Nucleic acids

*Heavy chain full length nucleic acid* (signal sequence-FR1-CDR1-FR2-CDR2-FR3-CDR3-FR4) 
ATGTACTTCAGCATCAGCTTGTTTTTCCTTGTGCTCATTTTAAAAAGTGTCCAGTGTGA
GGTACAGCTGGTGGAGTCTGGAGGAGGCTTAGTGCAGCCTGGAAAGTCCCTGAAAC
TCTCCTGTTCAGCCTCTGGATTCACATTCAGTAGGTATGGCATGCACTGGATTCGCC
AAGCTCCAGGAAAGGGGCTAGATTGGGTTGCATACATTACTCGTAGCAGCGATACAG
TCTATGCAGACGCTGTGAAGGGCCGGTTCACCATCTCCAGAGACAATGCAAAGAGC
ACCCTGTACCTGCAACTCAACAGTCTGAAGTCTGAAGACACTGCCATCTATTACTGTG
CAAGTCACTTTGCTACAGTGGAAAGTTACTGGGGCCAAGGAGTCATGGTCACAGTCT
CCTCA (SEQ ID NO: 17)
*Light chain full length nucleic acid* (signal sequence-FR1-CDR1-FR2-CDR2-FR3-CDR3-FR4)

ATGAGTCCTGTCCAGTCCCTGTTTTTATTATTGCTTTGGATTCTGGGAACCAAAGGTG
ATGTTGTGCTGACCCAGACTCCATCCATATTGTCTGTTACCGTTGGACAATCGGTCTC
CATCTCTTGCAGGTCAAGTCAGAGTCTCTTAGATAGTGATGGAAACACCTATTTATAT
TGGTTCCTACAGAGGCCAGGCCAGTCTCCACAGCGTCTAATTTATTTGGTATCCAAC
CTGGGATCTGGGGCCCCCAACAGGTTCAGTGGCAGTGGGTCAGGAACAGATTTCAC
ACTCAAAATCAGTGGAGTGGAGGCTGAGGATTTGGGAGTTTATTACTGCATGCAAGC
TACCCATGCTCCGTGGACGTTCGGTGGAGGTACCAAGCTGGAATTGAAA (SEQ ID
NO: 18).

The most promising antibody was selected for further testing. *In silico* modeling of the binding between the favored antibody (further one called eCyPA-mAb, extracellular anti-CyPA antibody) demonstrated strong interaction between the antibody (in green ribbons and brownish carbons) and the antigen (in turquoise ribbons and white carbons) right at the EMMPRIN binding site at the TAKTE sequence of CyPA (Figure 2F).

To validate the activity of the auspicious eCyPA-mAb, HL-1 cells were treated with recombinant CyPA, and cells size was determined in the absence or presence of the antibody. CyPA-dependent increase in HL-1 cell size was dose-dependently attenuated in the presence of eCyPA-mAb compared to IgG control (P<0.001) (Figure 2G). Comparison to a commercially available EMMPRIN-blocking antibody showed analogous results (Figure 2H).

Thus, effects on cardiomyocytes hypertrophy could be attributed to extracellular CyPA and the interaction with its receptor EMMPRIN.

### Neutralizing extracellular CyPA preserves myocardial function in Ang II-treated mice

The inventors' *in vitro* testing of an antibody directed against eCyPA already showed promising impact on cardiomyocyte hypertrophy. To gain insights into in vivo cardiac effects, *ApoE*^{*-*/*-*} mice were stressed by continuous administration of Ang II and treated with eCyPA-mAb over 28 days. Administration of the eCyPA-mAb was strictly controlled by IgG isotype controls throughout both experimental conditions, and administration of Ang II was controlled using 0.9% NaCl solution (saline) as healthy control (Methods). Myocardial function was assessed longitudinally by comprehensive echocardiography in one-week intervals, followed by extensive analysis of cardiac tissue after harvesting of the *ApoE*^{*-*/*-*} mice hearts after 28 days (Figure 3A, B).

The inventors found that both the ejection fraction (EF) and fractional shortening (FS) gradually decreased in Ang II + IgG-treated mice. In Ang II + eCyPA-mAb-treated mice, however, myocardial dysfunction was significantly attenuated (Figure 3C). Additionally, while therapeutic treatment resulted in a stabilization of myocardial function after 21 days, Ang II + IgG-treated mice displayed a trend towards further deterioration of heart function. After four weeks of chronic heart damage, the decrease of both EF and FS was significantly more pronounced in IgG control mice compared to anti-CyPA treated mice (P<0.0001) (Figure 3D, E).

To further characterize myocardial dysfunction, the inventors performed speckle-tracking-based strain analyses on the hearts. Analysis of global strain parameters including global longitudinal and circumferential strain (GLS, GCS) revealed better contractility in anti-eCyPA-treated mice compared to the IgG control group (Figure 3F-I), while peak radial strain was not significantly altered. Interestingly, temporal resolution of the cardiac contraction cycle showed a delayed systolic peak contraction, together with an increased diastolic relaxation velocity in the Ang II + IgG group, which was almost normalized in mice receiving anti-eCyPA treatment (Figure 3J, K). Reduction of longitudinal strain was mainly found in the apical region of Ang II + IgG treated mice (Figure 3B, overlays). Taken together, the strain analyses revealed a decrease in stiffening of the myocardium in anti-eCyPA treated mice, resulting in better systolic contractility and diastolic relaxation compared to IgG control. Since the inventors' findings suggest that Ang II-induced myocardial stiffness can be mitigated by inhibition of extracellular CyPA, they correlated echocardiographic measurements with cardiac CyPA expression, quantified by immunohistochemical staining of the hearts. The inventors found CyPA expression to critically correlate with global heart function and strain analyses, indicating a strong association of CyPA expression and myocardial contractility and stiffness (Figure 3K and L).

### Myocardial hypertrophy and stiffening are promoted by extracellular CyPA in vitro and in vivo

Among the multitude of factors influencing cardiac performance, myocardial stiffness stands out as a pivotal determinant considering its obvious impact on a contractile organ. Clinical data demonstrated that enhanced left-ventricular myocardial stiffness could represent an early transitional state from a healthy heart to heart failure with preserved ejection fraction (HFpEF). Others found that myocardial stiffness is associated with poorer event-free survival in patients with hypertrophic cardiomyopathy. One major factor contributing to myocardial rigidity is cellular hypertrophy. The inventors evaluated myocardial hypertrophy macroscopically (Figure 4A), by echocardiographic assessment of the left ventricular (LV) mass (Figure 4B), and determination of LV mass to body weight (BW) ratios (Figure 4C). All aspects demonstrated a significant reduction in cardiac hypertrophy in mice under treatment with eCyPA-mAb. Hypertrophy was also investigated on cellular levels, by quantification of the cell sizes (Figure 4D). Here, the inventors found similar positive effects on cardiac hypertrophy as in their *in vitro* assays before (Figure 2G, H).

Together with the inventors' previous findings, these results clearly demonstrate that extracellular CyPA is a driving force of myocardial hypertrophy.

When cardiomyocytes grow, the cell scaffold needs to adapt to the new conditions. The inventors hypothesized that cardiomyocytes develop increased stiffness during this process. To investigate if eCyPA has direct impact on cellular stiffness, they used atomic force microscopy (AFM) to measure cell stiffness *in vitro* and ex *vivo* (Figure 4F, G, Methods). Strikingly, HL-1 cardiomyocytes after 48 hours of treatment with Ang II + IgG showed significantly increased stiffness compared to cells under treatment with Ang II + anti-CyPA (P=0.010). To break down stiffness effects on extracellular CyPA, the inventors also treated cells with CyPA + eCyPA-mAb or IgG control. Strikingly, cells were even stiffer under specific CyPA treatment compared to Ang II treatment, and effects on stiffness markedly diminished under eCyPA-mAb treatment (P<0.0001).

The inventors next translated their approach to measure stiffness to myocardial tissue derived from the animal model described before. *Ex vivo* stiffness measurements were performed by AFM on 30 µm thick cryosections mounted on glass slides. The inventors selected the transitional region from myocardium to fibrosis at the area of the interventricular septum, identified by an adjacent fibrosis staining (Figure 4I). The regions of interest were scanned with a size of 50 x 50 µm. Notably, stiffness was significantly enhanced in Ang II + IgG challenged mice, compared to those treated with Ang II + eCyPA-mAb (P<0.0001) (Figure 4J). Linear regression analysis between ex *vivo* stiffness and *in vivo* echocardiographic stiffness measurements by strain analysis revealed a strong correlation and furthermore underpinned a marked effect on heart function (Figure 4K).

Thus, extracellular CyPA modulates hypertrophy and tissue stiffening in Ang II- induced heart failure.

### Inhibition of extracellular CyPA attenuates Ang II-induced myocardial inflammation and fibrosis

Besides cellular mechanisms like hypertrophy and cell stiffness, there are also extracellular factors contributing to cardiac rigidity and hence impaired contractile function. Extracellular CyPA is an important chemoattractant for immunocompetent cells. While inflammation is an essential part of the healing process, chronic inflammation can foster myocardial remodeling and lead to the development of cardiac fibrosis, which can both impair heart function. Myocardium from Ang II-treated mice displayed enhanced infiltration of CD3+ T cells and Mac-3+ macrophages. The number of inflammatory cells was significantly reduced in anti-CyPA-treated mice compared to IgG controls (Figure 5A, B). However, overall inflammation was considerably less pronounced compared to sections of acute myocardial infarction or myocarditis.

Further, myocardial fibrosis was substantially mitigated by treatment with eCyPA-mAb compared to IgG control (P<0.0001), as verified by Picro-Sirius Red staining (Figure 5C). Most interestingly, myocardial scarring and hypertrophy were strongly associated with areas where extracellular CyPA expression was enhanced (Figure 5D). Spatial colocalization of extracellular CyPA accumulation with regions of adverse cardiac remodeling provides further evidence for the pathomechanistic role of secreted CyPA in the cardiac microenvironment. To study systemic effects of global neutralization of secreted CyPA, immunologically sensitive organs were analyzed both macroscopically and microscopically. No relevant alterations in organ size or indications of auto-inflammatory or immunosuppressive phenomena were found.

To investigate how strong the histologic findings correlate with echocardiographic measurements, the inventors furthermore correlated both entities using correlation. Strong correlations were predominately apparent in CyPA-positive area percentage vs. GCS (correlation coefficient = 0.773) and fibrosis vs. FS (correlation coefficient = - 0.781).

### Extracellular CyPA modulates biomolecular composition of fibrosis and myocardium

As the inventors' previous findings have underpinned the significance of both cellular and extracellular remodeling, the inventors aimed to further characterize the actual biomaterial of the heart that contributes to cardiac stiffness and impaired contractility. They recently developed a method for profound, sophisticated evaluation of the molecular composition of ECM based on Raman spectroscopy. This approach, called "RAMAN Spectromics", employs a holistic, comprehensive analysis of untargeted spectral data acquired by spatially-resolved Raman spectroscopy (Figure 6A). The imaging method provides label-free insights into the biochemical composition of a sample, by pointing a laser on the probe and measuring inelastically-backscattered photons of polarizable molecules. Hence, the methodology allows to gain a deeper insight into the molecular rearrangements in myocardium after cardiac damage.

Cardiac compartments were identified by unsupervised clustering analysis of untargeted Raman spectra (Figure 6B, C). Then the spectral fingerprints of fibrotic regions and myocardium between Ang II + IgG vs. Ang II + eCyPA-mAb treated mice (n=5) were compared (Figure 6D). Concerning biomolecular differences of myocardium, peak alterations between the experimental groups could be mainly attributed to a conformational change of the polypeptide backbone, referred to differences in β-sheets40 of myosin (893, 901 cm⁻¹), These observations were enhanced in mice treated with Ang II + IgG and align harmoniously with the inventors' previously published data, where they could show that myocardial remodeling is associated with changes in protein secondary structure towards β-sheets. Even more intriguing were the molecular alterations identified in collagen. Besides overall reduced collagen peaks (814, 940, 1249, 1660 cm⁻¹), the inventors found especially lower hydroxyproline assignments (800-1000 cm⁻¹ range, especially 880 cm⁻¹ and 920 cm⁻¹) in mice treated with Ang II and the antibody which neutralizes eCyPA. Further spectral peaks -helical fibrils by crosslinking (1269 cm⁻¹) in Ang II + IgG treated mice, while anti-CyPA treatment resulted in reduced hydrogen bonds of collagen (1249/1339 cm⁻¹). Moreover, it has been shown previously that collagen fibers under increased mechanical strain show higher 1249 cm⁻¹ bands, which the inventors could also find in the stiff myocardium derived from Ang II + IgG treated mice.

Thus, the data of the inventors indicate that extracellular CyPA is a major driver of Ang II-induced heart failure resulting from cardiac stiffening. Consequently, inhibition of extracellular CyPA is a potent strategy to prevent myocardial dysfunction and hypertrophy.

### 4. Conclusions

The major findings of the inventors are: i) eCyPA plays a critical role in the development of Ang II-induced heart failure in mice. ii) inhibition of extracellular CyPA by a neutralizing anti-CyPA monoclonal antibody significantly reduced Ang II-induced myocardial hypertrophy, dysfunction, tissue inflammation and fibrosis in the failing heart and iii) myocardial stiffness is critically promoted by eCyPA and contributes to the impairment of myocardial contractility. The findings of the inventors imply that targeting eCyPA is a novel and promising strategy in prevention and treatment of non-ischemic heart failure by reducing myocardial inflammation and extracellular remodeling.

In summary, the present disclosure provides valuable insights into the pathomechanistic aspects of extracellular CyPA and its contribution to myocardial stiffening and dysfunction in cardiac hypertrophy. The detrimental effects of extracellular CyPA on cardiac function can be attributed to its ability to interact with the ECM and activate proinflammatory and profibrotic pathways. The potential clinical implications of the findings of the inventors are significant. By targeting the extracellular accumulation of CyPA it is possible to reduce the pathological effects associated with cardiac hypertrophy and improve heart function in patients with non-ischemic heart failure.

## Claims

1. An antibody or fragment thereof specifically binding to extracellular cyclophilin A (eCyPA), **characterized in** comprising
- as heavy chain variable domain a CDR3 region, said CDR3 region has an amino acid sequence being at least 90% identical to SEQ ID NO: 1, and/or
- as light chain variable domain a CDR3 region, said CDR3 region has an amino acid sequence being at least 90% identical to SEQ ID NO: 2.

2. The antibody or fragment of claim 1, **characterized in that**
- the heavy chain variable domain comprises a CDR2 region, said CDR2 region has an amino acid sequence being at least 90% identical to SEQ ID NO: 3, and/or
- the light chain variable domain comprises a CDR2 region, said CDR2 region has an amino acid sequence being at least 90% identical to SEQ ID NO: 4.

3. The antibody or fragment of claim 1 or 2, **characterized in that**
- the heavy chain variable domain comprises a CDR1 region, said CDR1 region has an amino acid sequence being at least 90% identical to SEQ ID NO: 5, and/or
- the light chain variable domain comprises a CDR1 region, said CDR1 region has an amino acid sequence being at least 90% identical to SEQ ID NO: 6.

4. The antibody or fragment of any of claims 1-3, **characterized in** comprising
- the heavy chain variable domains
- CDR3 region having an amino acid sequence being at least 90% identical to said in SEQ ID NO: 1 and/or
- CDR2 region having an amino acid sequence being at least 90% identical to said in SEQ ID NO: 3 and/or
- CDR1 region having an amino acid sequence being at least 90% identical to said in SEQ ID NO: 5 and/or
- the light chain variable domains
- CDR3 region having an amino acid sequence being at least 90% identical to said in SEQ ID NO: 2 and/or
- CDR2 region having an amino acid sequence being at least 90% identical to said in SEQ ID NO: 4 and/or
- CDR1 region having an amino acid sequence being at least 90% identical to said in SEQ ID NO: 6.

5. The antibody of fragment thereof or any of the preceding claims comprising
- a heavy chain having an amino acid sequence being at least 90% identical to said in SEQ ID NO: 7, and/or
- a light chain having an amino acid sequence being at least 90% identical to said in SEQ ID: 8.

6. The antibody or fragment thereof of any of the preceding claims, **characterized in that** it specifically binds to human eCyPA.

7. An antibody or fragment thereof configured for a specific binding to eCyPA for use in the prophylaxis and/or treatment of a systemic disease, preferably an inflammatory systemic disease, further preferably a cardiovascular disease.

8. The antibody or fragment thereof of claim 7, which is the antibody or fragment thereof of any of claims 1-6.

9. The antibody or fragment thereof of any of the preceding claims, which is a monoclonal antibody, preferably a humanized monoclonal antibody.

10. A pharmaceutical composition **characterized in** comprising an antibody or fragment thereof according to any of claims 1-9.

11. A nucleic acid encoding an antibody or fragment thereof according to any of claims 1-9.

12. A vector comprising the nucleic acid of claim 11 and, optionally, regulatory elements necessary for an expression in a prokaryotic and/or eukaryotic cell.

13. A prokaryotic or eukaryotic host cell comprising the vector of claim 12.

14. A method for the prophylaxis and/or treatment of a systemic disease, preferably an inflammatory systemic disease, further preferably a cardiovascular disease, in a living being, comprising the administration of a prophylactically and/or therapeutically effective amount of the antibody or fragment thereof according to any of claims 1-9, and/or the pharmaceutical composition of claim 10.

15. The method of claim 14, **characterized in that** the living being is a human being.
